# EUROPEAN PATENT SPECIFICATION

(11) **EP 2 828 778 B1**
(45) Date of publication and mention of the grant of the patent: **06.10.2021**
(21) Application number: 13720603.3
(22) Date of filing: 20.03.2013
(51) Int. Cl.: G16H 30/20, G16H 30/40, G16H 50/20, A61B 6/00

(54) **CLINICAL WORKSTATION INTEGRATING MEDICAL IMAGING AND BIOPSY DATA AND METHODS USING SAME**
KLINISCHER ARBEITSPLATZ MIT MEDIZINISCHEN BILDGEBUNGS- UND BIOPSIEDATEN SOWIE VERFAHREN DAMIT
STATION DE TRAVAIL CLINIQUE INTÉGRANT DES DONNÉES D'IMAGERIE MÉDICALE ET DE BIOPSIE ET PROCÉDÉS L'UTILISANT

(30) Priority: 21.03.2012 US 201261613640 P; 23.03.2012 EP 12160984
(43) Date of publication of application: 28.01.2015
(73) Proprietor: Koninklijke Philips N.V., 5656 AG Eindhoven (NL)
(72) Inventor: BANERJE, Nilanjana, NL-5656 AE Eindhoven (NL); KAMALAKARAN, Sitharthan, NL-5656 AE Eindhoven (NL); VARADAN, Vinay, NL-5656 AE Eindhoven (NL); JANEVSKI, Angel, NL-5656 AE Eindhoven (NL); DIMITROVA, Nevenka, NL-5656 AE Eindhoven (NL)
(74) Representative: Philips Intellectual Property & Standards
(86) International application number: PCT/IB2013/052224
(87) International publication number: WO 2013/140357

(56) References cited:
- WO-A1-2009/153723
- US-A1- 2006 258 933
- US-A1- 2008 221 929
- US-A1- 2009 118 640
- US-A1- 2010 087 756

## Description

The following relates to the medical arts, oncology arts, medical imaging arts, biopsy and pathology arts, and related arts.

Medical imaging is used in oncology and other medical areas to provide noninvasive or minimally invasive characterization of structural and/or functional aspects of a cancerous tumor, an organ containing metastasized cancerous cells, or other anatomical structures of interest. Various medical imaging modalities are known and exploited for their diverse imaging capabilities. Some imaging modalities used in various types of medical imaging include: magnetic resonance (MR) imaging; positron emission tomography (PET) imaging; single photon emission computed tomography (SPECT); transmission computed tomography (CT) or other transmission x-ray imaging techniques; and so forth. Some examples of imaging techniques include: static anatomical imaging (with or without an exogenous contrast agent); bolus imaging (where a vascular contrast agent bolus is administered and intake, washout, or other dynamics of the contrast agent is observed); functional MR imaging (fMRI, e.g. observing oxygenation state of brain tissue during functional activities); targeted contrast agent imaging (e.g., PET or SPECT imaging of a radioisotope that preferentially collects in a tissue type of interest); and so forth. Medical imaging also has some limited capabilities in terms of detecting macroscopic imaging biomarkers that are indicative of certain oncological conditions. For example, MRI biomarkers of tumor heterogeneity have been shown to be predictive of response to therapy. See O'Connor et al., "DCE-MRI biomarkers of tumour heterogeneity predict CRC liver metastasis shrinkage following bevacizumab and FOLFOX-6", British Journal of Cancer vol. 105 pages 139-145 (2011). However, it has been recognized that such imaging analyses can over-simplify the tumour biology, and could be advantageously augmented by other techniques such as microvascular heterogeneity measurements. *See Id.*

Invasive techniques employ a biopsy procedure to extract tissue that conventionally undergoes histopathology analysis (e.g., tissue sectioning, staining, and microscopic analysis). Histopathology enables unambiguous identification of tissue type, and in particular enables identification of whether a tumor is malignant (i.e., cancer) or benign. The biopsy sample can be acquired by completely removing the tumor or other anatomical feature of interest (incisional biopsy), but this approach is highly invasive and is generally not recommended unless there is a reasonable probability that the entire suspect mass can be removed. Alternatively, incisional or core biopsy techniques can be employed in which an interventional instrument removes only a portion of the tumor, e.g. a core sample acquired using a hollow needle or other interventional instrument. These approaches preserve the structural integrity of the sample. In needle aspiration biopsy the sample is drawn in a manner that does not preserve its structural integrity (e.g., well suited for acquiring a fluid sample that has no particular structure).

Molecular analyses have also been developed, which provide further tools for the oncologist. In these techniques, a biopsy sample is processed to assess genetic and/or proteomic information. Molecular tests have been developed for specific molecular markers, and for sets of molecular markers, the latter sometimes being performed in massively parallel fashion using a microarray or similar apparatus to concurrently assess dozens, hundreds, or more molecular markers using a single tissue sample. The molecular marker or set of markers is typically analyzed using a threshold or algorithm that outputs a clinically useful result respective to a specific medical condition. For example, a particular molecular marker or set of markers may be indicative of a particular type of cancer, or may measure the activity of a molecular signaling pathway associated with a known physical process, or so forth. Genetic sequencing techniques have also been developed that measure DNA and/or RNA sequences, sometimes up to and including the entire genome of the tissue (i.e., whole genome sequencing or WGS). Genetic sequencing techniques provide vast quantities of data for molecular analyses, which can be used in conventional molecular tests and/or in "discovery" analysis modalities in which the genetic data is searched in an effort to discover probative genetic features.

In a typical (simplified) oncological workflow, a screening test or physical examination reveals a suspected cancerous condition. Medical imaging is then performed to identify tumors or lesions in the suspect organ or tissue, or to provide anatomical characterization of a previously identified tumor (e.g., in the case of a suspicious breast lump identified by physical examination). The medical imaging is followed by a biopsy procedure that removes a sample of the imaged tumor, and histopathology and/or molecular tests are performed on the biopsy sample to determine whether the tumor is malignant and, if so, to assess the type of cancer. Treatment in the form of chemotherapy, brachytherapy, radiation therapy, or so forth is then applied based on the imaging and biopsy results. For example, if the biopsy indicates estrogen receptor (ER) positive breast cancer, then treatment that suppresses estrogen levels in the patient may be employed. These treatments are interlaced with further medical imaging sessions to assess whether the treatments are effective in reducing the tumor size. Further biopsies may also be performed to check for metastasis not detectable by the imaging.

In this workflow, the medical imaging and biopsy procedures provide complementary information, with the imaging providing spatial or anatomical information and the biopsy providing tissue identification and characterization. The imaging may also be used to provide some indirect tissue information - for example, bolus imaging can assess vascular density which can be suggestive of angiogenesis associated with cancer growth. Although indirect, such imaging information has the advantage of being minimally invasive (involving injection of a vascular contrast agent bolus) and providing spatially delineated information.

However, existing medical approaches do not effectively integrate the medical imaging and biopsy workflows. Conventionally, the information generated by medical imaging and biopsy, respectively, is generated separately and is combined manually by the physician who takes into account the collected imaging, histopathology, and/or molecular analysis information in assessing the patient case. At most, the imaging may be used to identify a biopsy site, and the skilled physician may manually guide some degree of workflow integration by ordering interleaved imaging and biopsy procedures over the course of the patient treatment in a manner that generates complementary information.

US 2006/258933 A1 discloses a method of defining a biological target for treatment in which image detectable markers are placed at and correlated to the location or locations of tissue biopsy. By obtaining pathological analysis of the biopsy tissue specimens and correlating the analysis to the corresponding marker locations, the definitive pathological analysis of the target tissue at the marker locations can be correlated to corresponding locations on a functional image of the target tissue. The correlation of the marker locations and pathology to the corresponding locations on a functional image can then be used to prescribe and apply modulated therapy to the target tissue.

US 2009/118640 A1 discloses systems for managing, planning and displaying the location of biopsy core sites across multiple same-patient visits or other stored biopsy plans to increase the confidence and accuracy of biopsy results.

WO 2009/153723 A1 discloses a method and system for performing biopsies can include an imaging system for obtaining diagnostic images of a target region; a tracking system; a probe having a deployable biopsy needle for performing a biopsy procedure where the tracking system generates tracking information for at least one of the probe and the biopsy needle; an ultrasound imaging system for obtaining ultrasound imaging of the target region; and a computer in communication with the tracking system, the imaging system and the ultrasound imaging system. The computer can register the tracking system with the imaging system. The computer transfers a marking of a biopsy site associated with the biopsy procedure from the ultrasound imaging to the diagnostic images based on the tracking information and the registration of the tracking system with the diagnostic images.

The following contemplates improved apparatuses and methods that overcome the aforementioned limitations and others.

According to one aspect, an imaging visualization workstation includes a graphical display device and an electronic data processor. The imaging visualization workstation is configured to perform a method including:
spatially registering a biopsy sample extracted from a medical subject with a medical image of the medical subject by identifying a region of the medical image of the medical subject that depicts a location of the medical subject from which the biopsy sample was extracted, by the electronic data processor; combining the medical image with a graphical representation of information generated from the biopsy sample to generate a combined image in which the graphical representation is spatially delineated based on the spatial registration of the biopsy sample by the electronic data processor; wherein the information generated from the biopsy sample includes lesion tissue growth rate, and the combining comprises: projecting, based on the size and shape of the lesion in the medical image combined with the lesion tissue growth rate, one or more future lesion boundaries; and wherein generating the combined image comprises generating the projected one or more future lesion boundaries superimposed onto the medical image; and displaying the combined image on the graphical display device of the imaging visualization workstation.

According to another aspect, a method comprises: spatially registering a biopsy sample extracted from a medical subject with a medical image of the medical subject by identifying a region of the medical image of the medical subject that depicts a location of the medical subject from which the biopsy sample was extracted; combining the medical image with a graphical representation of information generated from the biopsy sample to generate a combined image in which the graphical representation is spatially delineated based on the spatial registration of the biopsy sample; wherein the information generated from the biopsy sample includes lesion tissue growth rate, and the combining comprises: projecting, based on the size and shape of the lesion in the medical image combined with the lesion tissue growth rate, one or more future lesion boundaries; and wherein generating the combined image comprises generating the projected one or more future lesion boundaries superimposed onto the medical image; and displaying the combined image on a graphical display device.

According to another aspect, a non-transitory storage medium stores instructions executable by an electronic data processing device to perform operations including (i) spatially registering a biopsy sample extracted from a medical subject with a medical image of the medical subject by identifying a region of the medical image of the medical subject that depicts a location of the medical subject from which the biopsy sample was extracted, (ii) combining the medical image with a graphical representation of information generated from the biopsy sample to generate a combined image in which the graphical representation is spatially delineated based on the spatial registration of the biopsy sample; wherein the information generated from the biopsy sample includes lesion tissue growth rate, and the combining comprises: projecting, based on the size and shape of the lesion in the medical image combined with the lesion tissue growth rate, one or more future lesion boundaries; and wherein generating the combined image comprises generating the projected one or more future lesion boundaries superimposed onto the medical image; and (iii) displaying the combined medical image on a graphical display device.

One advantage resides in providing synergistic integration of imaging and biopsy information.

Another advantage resides in providing spatial mapping of biopsy information based on imaging data.

Numerous additional advantages and benefits will become apparent to those of ordinary skill in the art upon reading the following detailed description.

The invention may take form in various components and arrangements of components, and in various process operations and arrangements of process operations. The drawings are only for the purpose of illustrating preferred embodiments and are not to be construed as limiting the invention.
- FIGURE 1: diagrammatically shows an oncology workflow including an imaging visualization workstation that integrates medical images and information obtained by processing biopsy samples.
- FIGURE 2: diagrammatically shows illustrative extraction of biopsy samples from two lesions of a breast that are known or suspected to be malignant.
- FIGURES 3-5: diagrammatically show three alternative graphical display configurations that each combine a medical image of the two breast lesions of FIGURE 2 with a graphical representation of antigen Ki-67 level values obtained from analysis of biopsy samples extracted from the two lesions.
- FIGURE 6: diagrammatically shows a biopsy core obtained using an interventional instrument having a hollow needle for extracting the biopsy core.
- FIGURE 7: diagrammatically shows the lesion from which the biopsy core of FIGURE 6 is extracted, with a dashed line indicating the biopsy needle trajectory. Also indicated is the length (L_{core}) of the biopsy core.
- FIGURE 8: diagrammatically shows spatial registration of the biopsy core of FIGURE 6 in the lesion of FIGURE 7, where the spatial registration utilizes the lesion boundary identifiable in the biopsy core as a reference feature.
- FIGURE 9: shows a flow chart of another approach for spatial registering a biopsy sample with a medical image, which makes use of an interventional image showing the interventional instrument at the tissue extraction position.
- FIGURE 10: shows a flow chart of another approach for spatial registering a biopsy sample with a medical image, which makes use of biopsy scar that is visible in the medical image.
- FIGURE 11: diagrammatically shows allocation of contiguous regions of the lesion of FIGURE 7 to the biopsy samples sectioned from the biopsy core of FIGURE 6. The allocation employs an assumption of approximate spherical symmetry of the lesion.
- FIGURE 12: diagrammatically shows an allocation of a lesion region comprising multiple discontinuous portions of the lesion to the biopsy sample S₂ sectioned from the biopsy core of FIGURE 6.
- FIGURE 13: diagrammatically shows a combined image comprising the medical image with the lesion region of multiple discontinuous portions of FIGURE 12 false colored or highlighted based on information obtained from biopsy sample S₂.
- FIGURE 14: diagrammatically shows a combined image comprising the medical image overlaid with a chemotherapy access map computed based on microvasculature information obtained from biopsy samples and the size and shape of the lesion as depicted in the medical image.
- FIGURE 15: diagrammatically shows a combined image comprising the medical image rendered at low intensity or "grayed out" and overlaid with projected tumor sizes computed based on combination of lesion tissue growth rate estimated from biopsy samples and the present size and shape of the lesion as shown by the medical image.

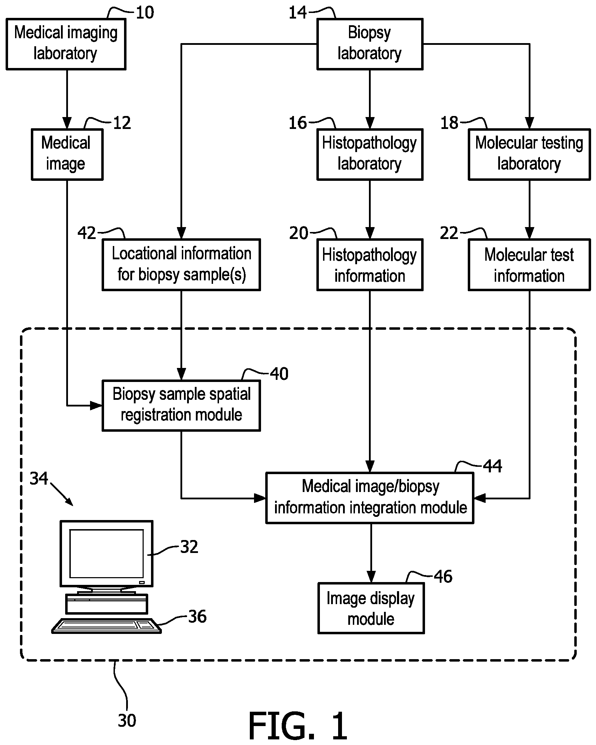

With reference to FIGURE 1, a medical imaging laboratory **10** acquires a medical image **12** of a medical subject using a suitable imaging modality such as magnetic resonance (MR) imaging, positron emission tomography (PET) imaging, single photon emission computed tomography (SPECT), transmission computed tomography (CT) or other transmission x-ray imaging techniques, or so forth. The medical image **12** may be two-dimensional, three-dimensional, or four-dimensional (e.g., a cinematic or CINE sequence tracking uptake and/or washout of a vascular bolus injection).

As used herein, the term "medical subject" encompasses hospital patients, out-patients (e.g., patients not admitted to the hospital but under medical care), clinical study subjects, medical screening subjects, veterinary subjects, and so forth. The medical image **12** is designed to elicit relevant information about a medical condition of the medical subject. In the illustrative case of an oncology medical subject, the medical image **12** is typically designed to elicit structural and/or functional information about a lesion that is suspected or known to be a malignant tumor. In some suitable embodiments the MR modality is used in conjunction with a vascular magnetic contrast agent to obtain functional information about uptake and washout to/from the lesion. Such measurements assess the vascular density and activity of the lesion which is probative of the growth rate of the cancer (since a fast-growing tumor requires an efficient blood supply). The sharpness of the boundary of high vascular activity can also be probative of the presence/absence and extent of metastasis. Additionally or alternatively, the MR imaging may be used to provide anatomical information about the size, shape, and other structural characteristics of the tumor.

For simplicity, reference is made herein to the illustrative medical image **12.** However, it is to be understood that typically multiple images may be acquired of the medical subject, using different imaging conditions or sequences (e.g., functional sequences and static anatomical images). Additionally, images may be acquired using two or more modalities. For example, in addition to MR imaging a nuclear imaging technique such as SPECT or PET may be used in conjunction with a tissue-specific radiopharmaceutical tracer in order to assess the presence and distribution of certain type of tissue.

In parallel with the imaging studies, the medical subject also visits a biopsy laboratory **14,** where a biopsy procedure is performed in order to extract a tissue sample. The biopsy procedure can be a surgical procedure (incisional biopsy) or can employ a biopsy needle or other interventional instrument. In some embodiments employing an interventional instrument, the biopsy procedure uses a hollow needle that is pushed into the tumor to extract a biopsy "core" whose dimensions correspond to a hollow interior of the biopsy needle. In this approach the structural integrity of the sample is preserved. In other embodiments, the biopsy procedure is a needle aspiration biopsy procedure typically used to extract a fluid or gelatinous tissue sample having limited structural integrity. The resulting extracted tissue is processed to generate one or more biopsy samples for analysis. In the case of a biopsy core or incised tissue sample, this typically entails sectioning the extracted tissue so as to generate a number of biopsy samples for analysis. For example, a biopsy core can be sectioned at multiple placed along the core so as to generate a line of biopsy samples extending along the length of the biopsy core (i.e., along the length of the biopsy needle as placed during tissue extraction). By pressing the needle into the tumor, the resulting line of biopsy samples are acquired at a range of depths into the tumor, which can be useful for assessing any inhomogeneity of the tumor.

The biopsy procedure targets a particular anatomical structure, typically a lesion suspected or known to be malignant in the case of an oncology subject. The lesion may be targeted using various sources of information. If the lesion can be identified externally (e.g., a breast lump suspected of being breast cancer) then targeting the lesion is straightforward. For other lesions, medical images acquired by the medical imaging laboratory **10** may be transferred to the biopsy laboratory **14** and used to target the lesion. However, this approach can be problematic due to difficulty in spatially registering the medical subject with the medical images that may have been acquired days earlier. In some cases, the biopsy laboratory **14** may have an imaging system available to generate current images. In another approach, the biopsy laboratory **14** has an interventional imaging system and the biopsy procedure is an image-guided procedure. In this approach the medical subject is imaged as the interventional instrument is inserted into the patient, and the imaging system enables medical personnel to observe the lesion and the inserted biopsy needle tip simultaneously, enabling precise placement of the needle tip into the lesion.

The biopsy samples generated by the biopsy laboratory **14** are sent to one or more analysis laboratories, such as an illustrative histopathology laboratory **16** and an illustrative molecular testing laboratory **18.** These laboratories **16, 18** process the biopsy sample or samples to generate biopsy information, that is, information obtained from the biopsy sample or samples. The histopathology laboratory **16** performs histopathology analyses which typically entail preparing microscope slides containing tissue that is stained by a probative staining agent and visually examining the stained tissue. Based on the staining characteristics, cell morphologies, and other visually perceived information, various forms of histopathology information **20** are generated. For example, by such procedures a skilled histopathologist can readily determine whether the tissue is malignant or benign and, if malignant, may provide information as to the type of cancer, the malignant/benign cellular ratio in the sample, and other cancer characterization useful to the oncologist.

The molecular testing laboratory **18** performs genetic and/or proteomic analyses on the biopsy sample or samples. The analysis may be targeted, e.g. using a microarray or other assay to determine the levels of a specific set of molecular markers followed by computing a result using these levels as inputs. The result may, for example, indicate the type of cancer, activity levels of relevant molecular signaling pathways or networks, or so forth. Additionally or alternatively, a genetic sequencing technique may be applied to measure DNA and/or RNA sequences, optionally up to and including the entire genome of the tissue (i.e., whole genome sequencing or WGS, typically defined as sequencing 95% or more of the entire genome). In some embodiments it is contemplated to sequence DNA or RNA from individual cells, so as (by way of illustrative example) to provide separate genetic profiles for benign and malignant tissues within the tumor. *See, e.g.* Navin et al., "Tumour evolution inferred by single-cell sequencing", Nature vol. 472 pp. 90-95 (2011). The sequencing dataset generated in WGS or the like is massive, and can be used in conventional molecular tests and/or in "discovery" analysis modalities in which the genetic data is searched in an effort to discover probative genetic features. The output of the molecular testing laboratory **18** is molecular test information **22.**

Thus, after completion of the testing the oncologist is provided with medical images **12** and biopsy information such as histopathology information **20** and/or molecular test information **22.** In some cases only the histopathology information **20** is generate, whereas in other cases only the molecular test information **22** is generated, whereas in still other cases both histopathology information **20** and molecular test information **22** are generated. It is also to be understood that these various tests may be ongoing - for example, the oncology patient may undergo an initial imaging and biopsy sequence in order to assess the initial (i.e., baseline) state of the cancer and to enable the oncologist to design a treatment regimen, possibly including one or more procedures such as chemotherapy, brachytherapy, radiation therapy, or so forth. At intervals during the treatment further imaging and/or biopsy assessments may be performed to assess the effectiveness of the treatment and to enable the treatment to be adjusted. It is still further to be understood that the illustrative laboratory topology of FIGURE 1, in which there are separate imaging, biopsy, histopathology, and molecular testing laboratories **10, 14, 16, 18** is merely an illustrative example, and other laboratory topologies can be employed. For example, a single laboratory may perform both histopathology and molecular testing, and in some cases that laboratory may also perform the biopsies. On the other hand, in some embodiments the biopsy laboratory may prepare the histopathology slides, and the histopathology laboratory may include only a few microscopes operated by a skilled histopathologist. As another example, the medical imaging and biopsy laboratories **10, 14** may be combined, as may be useful in order to provide skilled radiologists to operate interventional imaging systems used during image guided biopsy procedures.

Conventionally, the medical images **12** and the biopsy information **20, 22** are processed separately. For example, the radiologist acquires medical images as ordered by the oncologist, and may perform some analysis such as contouring features of interest. The resulting medical images are then passed onto the oncologist for review. Similarly, the histopathologist or other laboratory workers generate the biopsy information **20, 22** in accord with orders from the oncologist, and the biopsy information **20, 22** is also forwarded to the oncologist for review. The oncologist then considers this influx of data in diagnosing the cancer and designing and monitoring a treatment regimen.

With continuing reference to FIGURE 1, it is disclosed herein to improve upon this conventional approach by providing an imaging visualization workstation **30** that is configured to perform a method including: spatially registering a biopsy sample extracted from the medical subject with a medical image of the medical subject; combining the medical image with a graphical representation of information generated from the biopsy sample to generate a combined image in which the graphical representation is spatially delineated based on the spatial registration of the biopsy sample; and displaying the combined image on a graphical display device **32** of the imaging visualization workstation. The illustrative workstation **30** is embodied by a computer **34** that includes an electronic data processor (not shown) and suitable user input devices such as an illustrative keyboard **36,** a mouse, or so forth. More generally, the imaging visualization workstation **30** may comprise: a notebook computer; a desktop computer; a mobile device such as a smartphone, tablet computer, persona data assistant (PDA), or the like; a network server computer accessible via the Internet and/or a local wired/wireless data network; various combinations thereof; or so forth. While the illustrative display device **32** is a single screen, more generally the display device may comprise two or more screens or display devices, e.g. displaying the same content (mirroring) or displaying different content (e.g., so that a "virtual desktop" extends across the two or more display devices). The display device may also comprise a printer or other electronic marking engine that generates a permanent (monochrome or color) image on paper or another print medium. Further, the electronic data processor of the imaging visualization workstation may be local, e.g. a single-core or multi-core microprocessor and associated electronic components disposed inside the case of a personal computer, desktop computer, tablet computer, or the like, or may be embodied as a distributed electronic data processor, e.g. the workstation may comprise a "dumb terminal" connected via wired and/or wireless link with a network server that performs the electronic data processing.

The disclosed techniques for seamless integration of medical images and biopsy information are also suitably embodied as a non-transitory storage medium storing instructions executable by the illustrative computer **34** or other electronic data processing device to perform the disclosed processing and visualizations. The non-transitory storage medium storing the executable instructions may, for example, include: a hard disk drive or other magnetic storage medium; an optical disk or other optical storage medium; a flash memory, random access memory (RAM), read-only memory (ROM), or other electronic storage medium; or so forth.

With continuing reference to FIGURE 1, in order to integrate biopsy information with medical images the imaging visualization workstation **30** implements a biopsy sample registration module **40** that spatially registers each biopsy sample extracted from the medical subject with the medical image of the medical subject. The spatial registration utilizes locational information for biopsy samples **42** provided by the biopsy laboratory **14.** This information can be varied, and thus provides varying levels of spatial registration (i.e., varying levels of spatial localization of the biopsy sample respective to the imaged area). In some embodiments, the localization information **42** merely identifies from which lesion the biopsy sample was extracted. In this case the biopsy sample registration module **40** spatially registers the biopsy sample by associating it with the lesion from which it was extracted. In other embodiments the localization information **42** is more detailed, for example optionally including an interventional image that was acquired of the interventional instrument positioned to obtain the biopsy sample. In such cases, the spatial registration of the biopsy sample can be more precise. After spatial registration, a medical image/biopsy information integration module **44** implemented by the workstation **30** combines the medical image **12** with a graphical representation of information **20, 22** generated from the biopsy sample to generate a combined image in which the graphical representation is spatially delineated based on the spatial registration of the biopsy sample. An image display module **46** implemented by the workstation **30** displays the combined image on the display device **32.**

This approach provides an integrated display that enables the oncologist to consider the imaging and biopsy information concurrently, as an integrated unit. Moreover, as disclosed in illustrative examples herein, this combination can synergistically enhance the information above and beyond that provided by either imaging alone or biopsy alone. For example, the more detailed spatial information provided by the imaging can be leveraged to extend the spatial content of the biopsy information. Conversely, the biopsy information can be used to resolve an apparently homogeneous lesion into heterogeneous components.

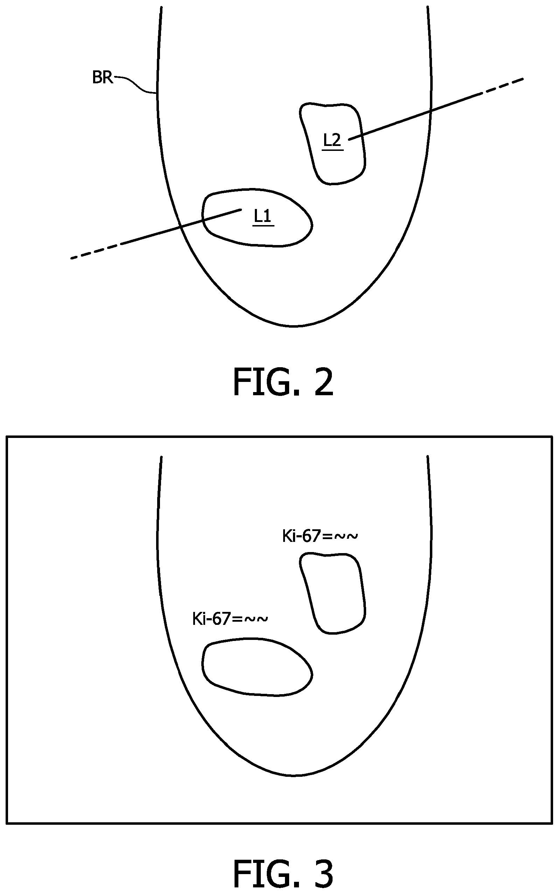

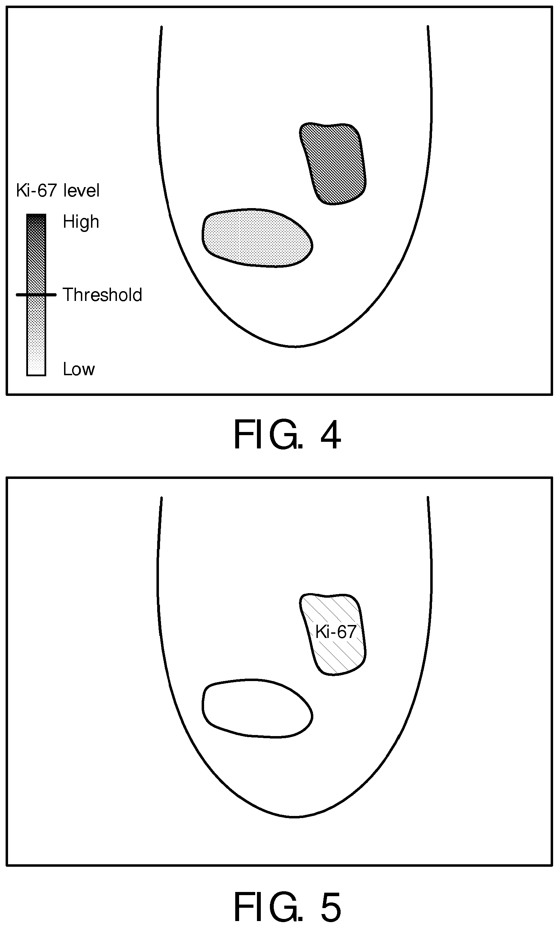

With continuing reference to FIGURE 1 and with further reference to FIGURES 2-5, some illustrative examples are set forth in which the locational information **42** is limited to identifying the lesion from which the biopsy sample is extracted. In this case, the spatial registration module **40** associates the biopsy sample with a depiction of the lesion in the medical image **12.** This is straightforward since the radiologist typically contours or otherwise segments the lesion or lesions of interest, and the locational information **42** provided by the biopsy laboratory includes identification of the lesion from which the biopsy sample was extracted. For example, FIGURE 2 shows a breast **BR** with two lesions **LI, L2.** A biopsy procedure collecting biopsy samples from both lesions is diagrammatically indicated by interventional instrument tips shown sampling each lesion indicated (diagrammatically by a line representing a biopsy needle). In the illustrative example, molecular testing and/or histopathology is applied to the biopsy sample of each lesion **LI, L2** to determine the expression level of the antigen Ki-67 in the respective lesions. Ki-67 is a cell proliferation marker that is associated with cancer proliferation. The Ki-67 level can therefore be expected to be indicative of the aggressiveness of the cancer, that is, whether and how fast it spreads or metastasizes. FIGURE 3 shows an example of a medical image showing the lesions **LI, L2** (which are not labeled in FIGURE 3 for clarity, but compare with FIGURE 2) and showing the measured Ki-67 expression level as a graphical representation in which each lesion is annotated numerically at a position proximate to the lesion (specifically, above the lesion in the example of FIGURE 3 using the textual annotation "Ki-67=∼∼∼∼" where "∼∼∼∼" generically denotes the quantitative Ki-67 expression level. FIGURE 4 shows another graphical representation in which the Ki-67 level for each lesion determined by biopsy is indicated by false coloring or highlighting. In false coloring, a color scale is used to indicate the Ki-67 level (for example, red may indicate the highest Ki-67 level on the scale and blue may indicate the lowest Ki-67 level on the scale). In highlighting, the intensity or texture of the lesion is set to indicate the Ki-67 level. Highlighting is useful if the graphical display device **32** is a monochrome display device. The color or highlight is determined by the information generated from the biopsy sample (namely the Ki-67 expression level in this example). The image of FIGURE 4 also includes a scale legend in the lower left-hand corner that indicates the correspondence between the false color or highlight and the Ki-67 expression level. The scale legend also shows a threshold indicating a Ki-67 expression level above which the lesion is deemed to be suspected cancer or cancer type. As seen in FIGURE 4, lesion **L2** (again, as labeled in FIGURE 2) has a substantially higher Ki-67 expression level than lesion **L1.** FIGURE 5 shows yet another graphical representation in which false coloring or highlighting is applied in a binary manner, that is, any lesion that has a Ki-67 expression level above the threshold for concern as indicated by biopsy information (namely lesion **L2** in this example) is false colored or highlighted while any lesion that is does not have a Ki-67 expression level above the threshold is not false-colored or highlighted. This graphical representation provides less information compared with the approaches of FIGURES 3 and 4, but is more likely to attract the oncologist's attention to the high Ki-67 expression level in the lesion **L2.**

A more general example of operation of a system in which the biopsy sample is spatially registered to (i.e. associated with) a lesion is as follows. The oncologist loads the medical image. In the imaging data, a lesion *L* is characterized by an image features vector *P_{I}(L)=*[*I₁, I₂,* ..., *I_{f}*]^{T} (where there are *f* imaging features *I₁*, *...,I_{f}* in the vector, and the superscript "T" denotes transpose). The image is suitably downloaded from a Picture Archiving and Communication System (PACS). For the same patient and the same lesion, the oncologist loads molecular test results *P_{M}(L)=* [*M₁, M₂,* ..., *M_{g}*]^{T} (where there are g molecular test results *M₁, ...,M_{g}* in the vector, and the superscript "T" again denotes transpose). It should be noted that the molecular test results vector *P_{M}(L)* may be a subset of the total set of molecular tests performed on the biopsy sample or samples extracted from lesion *L.* The correlation of the two features vectors *P_{I}(L)* and *P_{M}(L)* is provided by the spatial registration module **40.** In some embodiments, when the medical image is displayed and the oncologist selects a particular lesion *L* (for example, by clicking on the lesion *L* using a mouse), the contents of the feature vectors *P_{I}(L)* and *P_{M}(L)* are queried and presented as a pop-up table or other suitable representation.

Another, more specific, example of operation of a system in which the biopsy sample is spatially registered to (i.e. associated with) a lesion is as follows. This example integrates an imaging feature, namely the blooming index (BI) measured by MRI using a vascular bolus injection, with a molecular test result, namely the vascular endothelial growth factor (VEGF) level information. Blooming is a characterization of the margin of a mass over time. Blooming describes a fast-enhancing mass with initially sharply delineated borders that become un-sharp several minutes after the bolus injection of a contrast agent. The degree to which the margin blurs into the background indicates the amount of blooming present. The degree of blooming is expected to positively correlate with angiogenesis. Early signs of blooming can be evaluated by detecting low-level peripheral enhancement on the subtraction image derived from a post-contrast image and the pre-contrast image. Using a single, pre-configured post-contrast image that occurs at (or near) peak enhancement reduces adverse effects of motion and other time-varying image artifacts. A blooming index (BI) is computed as integral values in the range of [0, 25]. *See* Penn et al., "Morphologic Blooming in Breast MRI as a Characterization of Margin for Discriminating Benign from Malignant Lesions," Acad Radiol 2006; 13:1344-1354; Fisher et al., "Is the 'blooming sign' a promising additional tool to determine malignancy in MR mammography?" Eur Radiol (2004) 14:394-401). A higher BI value indicates a greater amount or degree of bloom. Under this imaging test, a lesion is considered suspicious if BI≥7. The blooming index is evaluated on a slice-by-slice basis in the primary plane of acquisition on all possible lesions larger than a threshold size (e.g., larger than 8mm). When suspicious possible lesions overlap on adjacent slices, they are merged into three-dimensional clusters.

Vascular endothelial growth factor (VEGF) is a signal protein produced by cells that stimulates vasculogenesis and angiogenesis. It is part of the system that restores the oxygen supply to tissues when blood circulation is inadequate. The normal function of VEGF is to create new blood vessels during embryonic development, new blood vessels after injury, muscle following exercise, and new vessels (collateral circulation) to bypass blocked vessels. However, when VEGF is overexpressed, it can contribute to disease. Solid cancers cannot grow beyond a limited size without an adequate blood supply; cancers that can express VEGF are able to grow and metastasize. Overexpression of VEGF can cause vascular disease in the retina of the eye and other parts of the body. Drugs such as bevacizumab can inhibit VEGF and control or slow those diseases.

Since both high blooming factor (BI) and VEGF overexpression are linked to high angiogenesis rate associated with some cancers, it is reasonable to expect the BI and VEGF level to have some correlation. By employing a combined image display such as those shown in FIGURES 3-5 (but with the annotated value, false coloration, or highlighting being associated with VEGF and blooming index, e.g. using numeric values for VEGF and false coloration for BI or vice versa) the oncologist can readily assess both metrics to provide confirmation of a high angiogenesis rate suggestive of high cancer growth. The VEGF expression level can be assessed based on the biopsy using various techniques. In a histopathology approach, a biopsy sample is stained and examined under a microscope by a trained pathologist to assess the VEGF quantitatively. Alternatively, substantially equivalent information can be obtained from genetic sequencing data. In one approach, a readout of all the genes in the VEGF pathway is obtained from sequencing data generated from a biopsy sample, and a correlation is performed between a vector of genomic (transcriptomic or methylation) features against a vector of imaging features (e.g. the blooming index as described above, and optionally other pertinent features such as tumor volume and/or shape). A correlation vector is generated instead of correlation value for each patient.

As another example, the imaging can correlate image texturing associated with cell adhesion with molecular markers associated with cell adhesion. One cell function is the binding of a cell to a surface, extracellular matrix or another cell using cell adhesion molecules. There are multiple genes (or, when expressed, proteins) involved such as selectins, integrins, and cadherins. Proper adhesion maintains multicellular structure, and disregulation of this function is expected to be linked to tumorigenesis. These molecules are also involved in signal transduction. On the biopsy side, genomic features can be assessed based on their role in adhesion. For example, a genomic vector can include the levels of all adhesion molecules. On the imaging side, different types of texture features are suitably assessed to identify correlations between certain sets of adhesion molecules and a resulting irregular texture in the imaged tumor. Conversely, if a clinician notices certain imaging features such as a particular texture type, this can be further investigated based on sequencing of tumor biopsy samples to generate molecular features associated with adhesion (e.g., expressed as a genomic vector as just described). In this example, the information generated from the biopsy sample (namely the genomic vector of adhesion-related genes or a result derived from that information, such as an activity level for an adhesion-related pathway or network) is suitably displayed numerically as per the example of FIGURE 3, and image enhancement such as false coloring or highlighting is suitably used to indicate areas of the image that exhibit the particular texture type under investigation. In this way, the clinician can readily assess whether there is a positive correlation between disregulation of the adhesion function as indicated by the textually represented biopsy information and image texturing indicated by highlighting or false coloration.

In the foregoing examples, the biopsy sample is spatially registered to (i.e. associated with) a lesion. However, if the available locational information **42** is sufficient, the spatial registration of the biopsy sample can be more finely grained, e.g. a biopsy sample can be associated with a particular portion of a lesion. Some examples of ways this can be done follow.

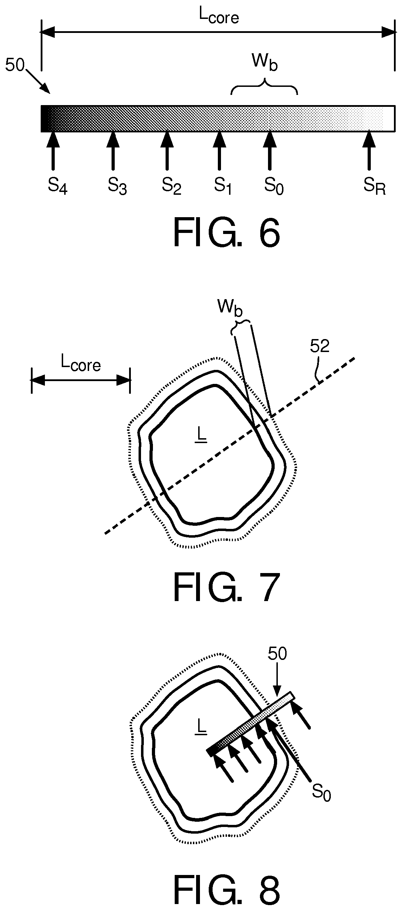

With reference to FIGURES 6-8, a biopsy core **50** obtained using a hollow biopsy needle is shown. The biopsy core **50** has length L_{core}, and six biopsy samples S₀, S₁, S₂, S₃, S₄, and S_{R} are generated by sectioning the biopsy core **50** as indicated in FIGURE 6. As shown in FIGURE 7, the biopsy core **50** is obtained by pressing the hollow biopsy needle into the lesion **L** along a trajectory **52.** The lesion **L** has a boundary that is not abrupt but rather has a finite transition of width W_{b}. (In some other examples, the lesion boundary may be abrupt). In performing the sectioning, the skilled technician examples the biopsy core **50** under a microscope and can thereby identify the transition of width W_{b}. Based on this observation, the technician performs sectioning to obtain a biopsy sample S₀ at the center of the boundary width W_{b} and obtains biopsy samples S₁, S₂, S₃, and S₄ at progressively larger depths into the lesion **L.** Additionally, as tissue from outside the lesion **L** is available, the technician obtains a reference biopsy sample S_{R}. The biopsy samples S_{R}, S₀, S₁, S₂, S₃, and S₄ thus form a line of biopsy samples. In this case, the locational information **42** of FIGURE 1 includes the information that sample S₀ is located at the boundary of the lesion **L** (thus providing a reference feature for the spatial registration), the known biopsy needle trajectory **52,** and the known length L_{core} of the biopsy core **50,** as well as the known locations of the biopsy samples S_{R}, S₀, S₁, S₂, S₃, and S₄ along that length. As shown in FIGURE 8, this information is sufficient to spatially register the biopsy core **50** and the biopsy samples S_{R}, S₀, S₁, S₂, S₃, and S₄ respective to a depiction of the lesion **L** in the medical image **12.**

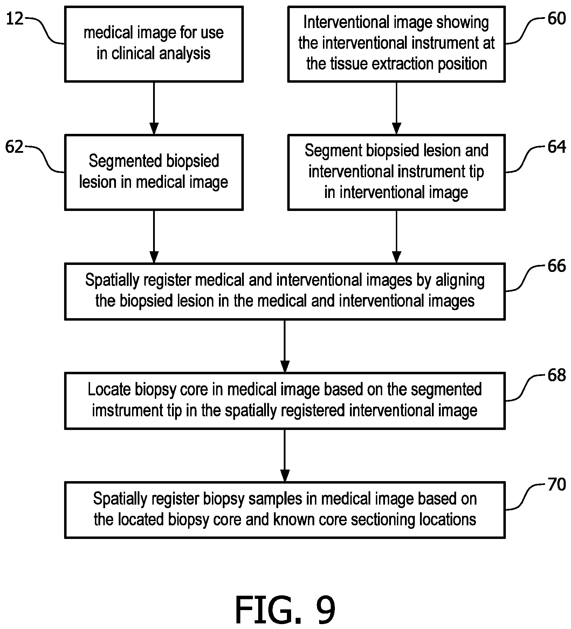

With reference to FIGURE 9, another approach for spatially registering a biopsy sample is described, which utilizes an interventional image. As already discussed, the medical image **12** is designed for use in clinical analysis, and as such is typically of high spatial resolution and/or employs a contrast mechanism (such as vascular contrast agent bolus injection) that yields probative information. In this example the biopsy sample is acquired by an image-guided biopsy procedure. The interventional imaging system employed in the image guidance is typically of relatively low resolution - it is sufficient to resolve the tip of the biopsy needle and the lesion with sufficient resolution to accurately position the needle tip in the lesion. Moreover, the interventional imaging typically does not employ special contrast mechanisms (although the use of a tracking micro-coil or other active or passive marker disposed on or in the tip of the interventional instrument is contemplated). Thus, the interventional images obtained during the image-guided biopsy procedure are usually of little or no clinical value, and are not normally utilized except for the image guidance. In the spatial registration approach of FIGURE 9, however, an interventional image **60** showing the interventional instrument at the tissue extraction position is supplied to the spatial registration module **40** as (at least part of) the locational information **42** (see FIGURE 1). In an operation **62** the depiction of the biopsied lesion in the medical image **12** is segmented, either manually (e.g., by manual contouring of the lesion) or automatically, or by a semi-automatic approach. In an operation **64** the interventional image **60** is similarly segmented to delineate the biopsied lesion and the interventional instrument tip. In an operation **66,** the medical image **12** and the interventional image **60** are spatially registered by aligning the segmented biopsied lesion in the two images. In an operation **68,** the biopsy core extracted by the hollow biopsy needle (e.g., the biopsy core **50** of FIGURE 6) is located in the medical image based on the segmented instrument tip in the spatially registered interventional image. (This is based on the recognition that the biopsy core **50** is disposed inside the hollow biopsy needle tip at the extraction position). In an operation **70,** the individual biopsy samples sectioned from the biopsy core **50** are spatially registered with the medical image **12** based on the located biopsy core and the known sampling locations (e.g., sampling locations S_{R}, S₀, S₁, S₂, S₃, S₄) along the line of the biopsy core.

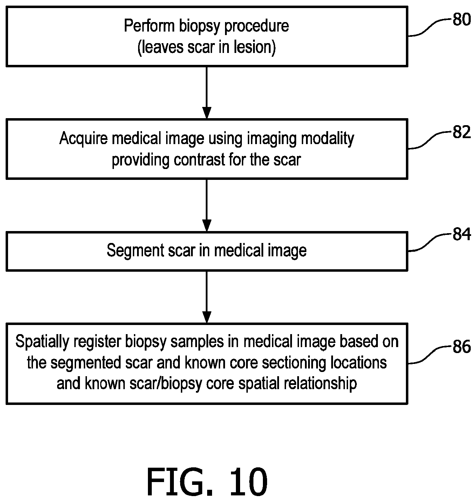

With reference to FIGURE 10, yet another approach for spatially registering a biopsy sample is described. This approach is appropriate if the biopsy is performed before the medical image **12** is acquired, and if the biopsy procedure leaves a biopsy scar in the lesion that is visible in the medical image **12.** In this case, the approach of FIGURE 10 is as follows. The biopsy procedure is performed first in an operation **80.** This biopsy procedure **80** leaves a biopsy scar in the lesion. In an operation **82** the medical image **12** is acquired, using an imaging modality for which the biopsy scar is visible. In an operation **84** the biopsy scar is segmented (again, using manual, automatic, or semi-automatic segmentation). In an operation **86,** the biopsy samples are spatially registered with the medical image **12** based on the segmented biopsy scar and known biopsy core section locations along the core (e.g., sampling locations S_{R}, S₀, S₁, S₂, S₃, S₄ of FIGURE 6) and a known spatial relationship between the biopsy scar and the biopsy core. This known relationship may be precisely one-to-one, i.e. the core precisely matching the imaged biopsy scar; however, it is alternatively contemplated to employ a spatial transform to accommodate shrinkage or other known scar distortion mechanisms.

The spatial registration approaches described with reference to FIGURES 6-10 provide finer-grained spatial localization of a biopsy sample to a region smaller than the lesion as a whole. However, the biopsy sample (e.g., biopsy samples S₁, S₂, S₃, S₄ of FIGURES 6-8) are only along a line and do not map out the volume of the lesion. However, in embodiments described herein the spatial imaging data provided by the medical image **12** is leveraged to enable the biopsy samples to provide characterization of volumetric regions of the lesion.

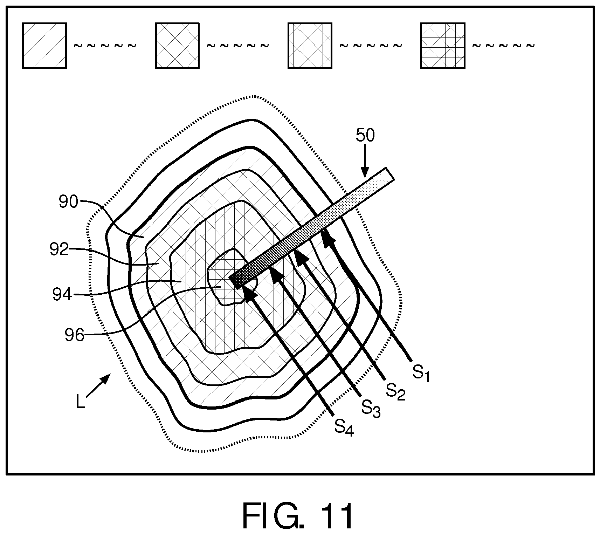

With reference to FIGURE 11, in one approach the lesion is assumed to have approximately spherical symmetry. Thus, the biopsy sample S₁ is assumed to be representative of an isocontour shell **90** corresponding to the depth of the biopsy sample S₁ in the lesion **L.** In the same way, the biopsy sample S₂ is assumed to be representative of an isocontour shell **92** inside the isocontour shell **90** and corresponding to the depth of the biopsy sample S₂ in the lesion **L.** The biopsy sample S₃ is assumed to be representative of an isocontour shell **94** inside the isocontour shell **92** and corresponding to the depth of the biopsy sample S₃ in the lesion **L.** Finally, the deepest biopsy sample S₄ is assumed to be representative of a central roughly spherical core **96** inside the isocontour shell **94.** The four regions **90, 92, 94, 96** defined in the lesion **L** are suitably false colored, highlighted, or labeled based on biopsy information obtained from the corresponding four biopsy samples S₁, S₂, S₃, S₄ sectioned from the biopsy core **50.** More generally, the image location containing the biopsy sample is extended to a contiguous (i.e. connected) region containing the biopsy sample based on suitable assumptions or spatial modeling of the lesion (in the case of FIGURE 11, the assumption is that the lesion **L** has approximately spherical symmetry where the deviation from perfect spherical symmetry follows the deviation of the boundary of the lesion **L** from perfect spherical symmetry). FIGURE 11 shows a legend at the top of the display identifying the meaning of the various false colors or highlights. FIGURE 11 also shows the biopsy core **50** and labels the four biopsy samples S₁, S₂, S₃, S₄ - however, these items would typically not be displayed to the oncologist.

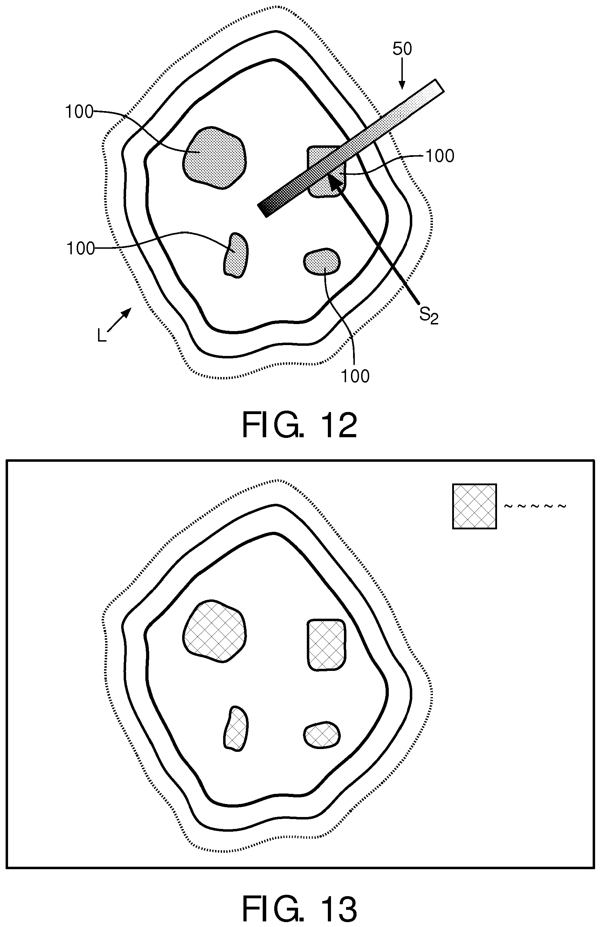

With reference to FIGURES 12 and 13, in another approach for extending the discrete biopsy samples across the lesion operates by (1) identifying an image characteristic at the location of the medical image spatially registered with the biopsy sample and (2) defining a region of the medical image associated with the biopsy sample as one or more portions of the medical image having the identified image characteristic. FIGURE 12 illustrates an example using the biopsy sample S₂ obtained from the biopsy core **50** of FIGURE 6. The image characteristic at the location of the medical image in this example is diagrammatically indicated by a darker contrast as compared with other areas of the lesion **L.** More generally, the image characteristic can be any image feature of interest that is present at the location of the biopsy sample S₂, such as a characteristic intensity level, a characteristic texturing or other micromorphology, a particular value for bolus intake measured in an image sequence, or so forth. To extend the range of the region associated with and containing the biopsy sample S₂, the integration module **44** searches the medical image **12** to identify other portions of the image that also exhibit this image characteristic (within a suitable tolerance). In the example of FIGURE 12, it is seen that there are four distinct portions or sub-regions **100** having the image characteristic of the location of the biopsy sample S₂. FIGURE 13 illustrates a modified version of the medical image **12** that includes the biopsy information from biopsy sample S₂ as a false coloration or highlighting (diagrammatically indicated by crosshatching in FIGURE 13) of the identified regions **100** associated with the biopsy sample S₂. The modified image of FIGURE 13 also includes a legend in the upper right corner identifying the significance of the false coloration or highlighting.

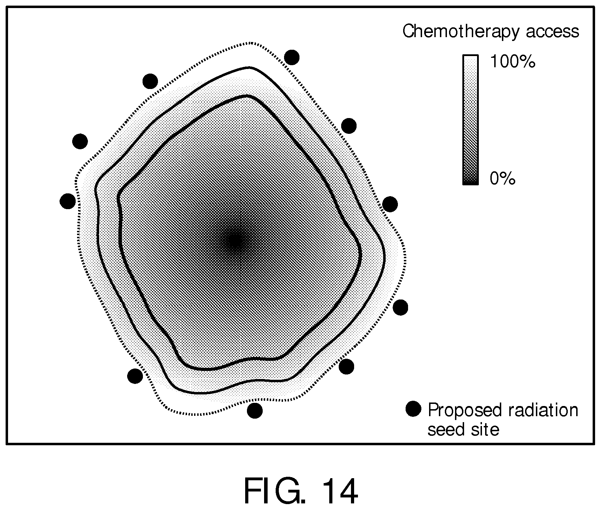

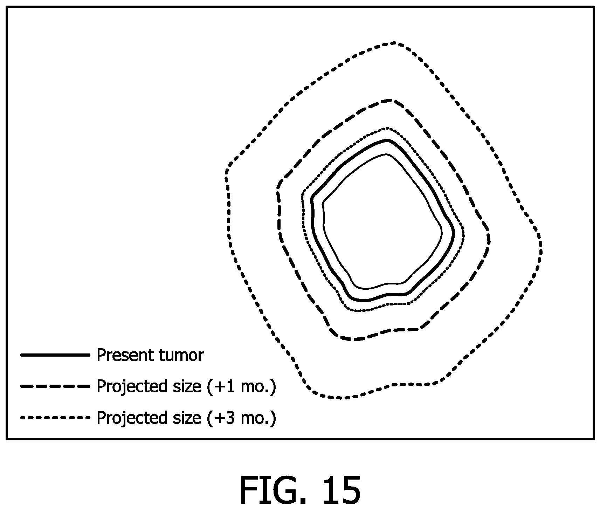

With reference to FIGURES 14 and 15, the information generated from the biopsy sample may comprise information generated from the biopsy sample and also from content of the medical image. Said another way, the information generated from the biopsy sample and displayed on the modified or combined image may constitute information generated by a mathematical or other combination of biopsy and imaging data. In the example of FIGURE 14, processing of biopsy samples is used to generate biopsy information that includes microvasculature information for lesion tissue. As already noted, the vascular endothelial growth factor (VEGF) signal protein is a molecular marker that is indicative of angiogenesis rate, i.e. higher VEGF expression typically correlates with a higher angiogenesis rate. Regions of higher angiogenesis rate may be expected to have higher microvasculature density. If the biopsy sample is large enough, a more direct approach for estimating the microvasculature information is to measure the microvasculature density for biopsy samples S₁, S₂, S₃, S₄ at different depths into the lesion **L** (again using the illustrative biopsy core of FIGURE 6 as an example). Assuming the generally spherical model of FIGURE 11, this enables estimation of the microvasculature density as a function of depth inside the lesion **L.** With this detailed microvasculature density information and the known structure of the lesion **L** as provided by the medical image **12,** a spatial map of chemotherapy accessibility to the lesion **L** can be estimated. Since it is assumed that the chemotherapy drug is delivered via the microvasculature, the map of chemotherapy accessibility can also be thought of (with suitable magnitude scaling) as a blood perfusion map for the lesion. FIGURE 14 diagrammatically illustrates a combined image comprising the estimated spatial map of chemotherapy accessibility to the lesion **L** superimposed onto the medical image **12.** In this example, it is seen that chemotherapy access is high for the outer portion of the lesion **L** and decreases deeper inside the lesion such that the innermost portion of the lesion **L** has the lowest chemotherapy access. Such a map could result from low microvascular density deep inside the lesion (where the tissue may be stagnant or even partially necrotic) and higher microvascular density near the boundary of the lesion **L** where the cancer is proliferating. FIGURE 14 also includes a scale legend in the upper right corner.

Another example, also depicted in FIGURE 14, applies to planning brachytherapy rather than chemotherapy. Brachytherapy entails implantation of radioactive seeds in or proximate to the tumor with the expectation that radiation released from the seeds over time will suppress the malignancy. In this case, relevant information obtained from the biopsy sample may include a radiation absorption characteristic of lesion tissue for radiation emitted by a brachytherapy radiation seed. For example, the radiation absorption characteristic may be quantified as a radiation absorption coefficient, an extinction coefficient for the radiation, a transmissivity coefficient for the radiation, or so forth. These values may be measured on the biopsy sample directly using suitable equipment, or alternatively may be determined from the tissue type and/or tissue density using a lookup table or the like. FIGURE 14 diagrammatically shows a combined image in which an optimal placement of brachytherapy radiation seeds respective to the lesion is shown superimposed on the medical image. The optimal placement is determined based on the depiction of the lesion in the medical image and the radiation absorption characteristic of lesion tissue. The optimal placement is chosen to provide a desired radiation exposure through the volume of the lesion, with the exposure at any particular location in the lesion being computed based on the proximity of the radiation seed(s) and the computed absorption (e.g., computed as an integral of the absorption characteristic over the space separating the radiation seed from the location where exposure is being computed). In FIGURE 14, the optimal placement of brachytherapy seeds is indicated by superimposing icons (namely filled circle icons) representing the radiation seeds.

For conciseness, FIGURE 14 depicts both the chemotherapy access map and the optimal placement of brachytherapy seeds. Of course, if only chemotherapy is contemplated then the brachytherapy seed placement overlay is suitably not computed or displayed, and likewise of only brachytherapy is contemplated then the chemotherapy access map is suitably not computed or displayed. In some embodiments both chemotherapy and brachytherapy may be planned, in which case the display shown in FIGURE 14 is suitable. If only brachytherapy is planned, then the chemotherapy access map may be replaced by a map of estimated radiation dosage density computed for the optimal placement of brachytherapy seeds and the radiation absorption characteristic determined from the biopsy sample. While two specific mapping examples have been mentioned (the chemotherapy access map shown in FIGURE 14, and a radiation dosage map suitably computed for the optimal radiation seed placement), the skilled artisan will readily recognize that these examples can be generalized to a procedure comprising: computing a map of a derived characteristic for the lesion wherein the derived characteristic depends upon the biopsy information and spatial location within the lesion; and displaying the map of the derived characteristic superimposed on the medical image.

FIGURE 15 illustrates another example, in which future lesion growth is projected based on a combination of lesion tissue growth rate estimated from biopsy samples and the present size and shape of the lesion as shown by the medical image **12.** VEGF expression level determined from the biopsy sample can again be used as a growth rate estimate. Other molecular markers indicative of cellular proliferation rate can additionally or alternatively be measured. Alternatively, histopathology comparison of biopsy sample S₀ (taken from the boundary of the lesion **L,** see FIGURE 6) and biopsy samples S₁, S₂, S₃, and/or S₄ (taken from inside the lesion **L)** can be used to more directly assess the rate of cellular proliferation at the lesion boundary. From the cellular proliferation rate obtained from the biopsy sample(s) and the current lesion boundary shown in the medical image **12,** one or more future lesion boundaries can be projected for various times in the future. FIGURE 15 shows a combined image showing the current lesion with its boundary (solid line, the current boundary being taken as the middle of the transition region of width Wb, see FIGURES 6 and 7) and projected future lesion boundaries for one month in the future (dashed line) and three months in the future (dotted line) superimposed onto the medical image **12.** In illustrative FIGURE 15, the medical image **12** is shown at reduced intensity, i.e. "grayed out" in FIGURE 15, so as to emphasize the projected lesion growth. A legend in the lower left corner identifies the line types.

It should be noted that the future growth estimates are for the cell proliferation rate estimated from the biopsy samples - this rate would not take into account any oncological therapy that is applied subsequent to the biopsy procedure. Thus, for example, if the biopsy procedure was performed before the therapy regimen was initiated then the projected lesion growth shown in FIGURE 15 represents the expected growth without therapy. A useful workflow would then be to initiate the therapy regimen and to acquire an image of the actual tumor at one month and three months in the future. Comparison of those images with the projected tumor sizes at one- and three-months shown in FIGURE 15 then would provide an effective graphical indication of the effectiveness of the therapy, e.g. if the lesion has grown but at a rate less than that projected in FIGURE 15, this may indicate the therapy has some effectiveness even though it is not actually causing shrinkage of the tumor (i.e., the therapy is causing the tumor to grow less rapidly).

The examples set forth herein are provided for illustrative purposes. The skilled artisan, upon reading and understanding the disclosure herein including the illustrative examples, can readily employ the disclosed imaging visualization workstation and/or the disclosed techniques for seamlessly integrating imaging and biopsy data to provide other synergistic configurations that are probative for various types of oncology applications as well as various other clinical and/or veterinary applications. For example, the disclosed techniques can readily be applied to cardiology so as to seamlessly integrate cardiac images (optionally employing a vascular contrast agent bolus to dynamically image blood flow through the heart and major vasculature) with information related to cardiac function obtained by processing biopsy samples, such as monitoring of natriuretic peptide levels (e.g., BNP, ANP, or so forth), in order to provide reinforcement or extension of the information obtained by either imaging or biopsy alone.

The invention has been described with reference to the preferred embodiments. Obviously, modifications and alterations will occur to others upon reading and understanding the preceding detailed description. It is intended that the invention be construed as including all such modifications and alterations insofar as they come within the scope of the appended claims.

## Claims

1. An apparatus comprising:
an imaging visualization workstation **(30)** including a graphical display device **(32)** and an electronic data processor, the imaging visualization workstation being configured to perform a method including:
spatially registering a biopsy sample (S_{R}, S₀, S₁, S₂, S₃, S₄) extracted from a medical subject with a medical image **(12)** of the medical subject by identifying a region of the medical image **(12)** of the medical subject that depicts a location of the medical subject from which the biopsy sample was extracted, by the electronic data processor,
combining the medical image with a graphical representation of information **(20, 22)** generated from the biopsy sample to generate a combined image in which the graphical representation is spatially delineated based on the spatial registration of the biopsy sample by the electronic data processor, **characterized in that** the information generated from the biopsy sample includes lesion tissue growth rate, and the combining comprises:
projecting, based on the size and shape of the lesion in the medical image combined with the lesion tissue growth rate, one or more future lesion boundaries; and
wherein generating the combined image comprises generating the projected one or more future lesion boundaries superimposed onto the medical image; and
displaying the combined image on the graphical display device of the imaging visualization workstation.

2. The apparatus of claim **1** wherein the biopsy sample is obtained from a lesion of the medical subject and wherein:
the spatially registering associates the biopsy sample with a depiction of the lesion in the medical image; and
the combined image associates the graphical representation of information generated from the biopsy sample with the depiction of the lesion in the medical image.

3. The apparatus of claim **2** wherein the combining comprises at least one of:
labeling the depiction of the lesion in the medical image with the information generated from the biopsy sample extracted from the lesion, and
false coloring or highlighting the depiction of the lesion in the medical image with a color or highlight determined by the information generated from the biopsy sample.

4. The apparatus of claim **1** wherein the biopsy sample comprises a section of extracted tissue, and the spatial registering comprises:
identifying a reference feature in the medical image; and
locating the extracted tissue in the medical image based on the reference feature.

5. The apparatus of claim **4** wherein the reference feature comprises a lesion boundary.

6. The apparatus of claim **4** wherein the reference feature comprises a biopsy scar.

7. The apparatus of claim **1** wherein the biopsy sample is obtained using an interventional instrument and an interventional image **(60)** is acquired of the interventional instrument positioned to obtain the biopsy sample, and the spatial registering comprises:
spatially registering the medical image and the interventional image; and
locating the biopsy sample in the medical image based on the spatially registered interventional image.

8. The apparatus of any one of claims **1-7** wherein the combining comprises:
defining a region **(90, 92, 94, 96, 100)** of the medical image **(12)** depicting a portion of the medical subject from which the biopsy sample was extracted according to the spatial registering; and
modifying the region of the medical image to delineate the region and to indicate the information generated from the biopsy sample.

9. The apparatus of claim **8** wherein the biopsy sample comprise a line of biopsy samples (S₀, S₁, S₂, S₃, S₄) extracted from different depths in a lesion and the defining comprises:
for each biopsy sample, defining the region **(90, 92 94, 96)** as an isocontour shell corresponding to the depth of the biopsy sample in the lesion within an outer shell that is based on the size and shape of the lesion and wherein the defined region is a contiguous region.

10. The apparatus of claim **8** wherein the defining comprises:
identifying an image characteristic at the location of the medical image **(12)** spatially registered with the biopsy sample; and
defining the region of the medical image as one or more portions **(100)** of the medical image having the identified image characteristic.

11. The apparatus of any one of claims **1-10** wherein the biopsy sample is obtained from a lesion of the medical subject, the information generated from the biopsy sample includes microvasculature information for lesion tissue, and the combining comprises:
estimating, based on structure of the lesion in the medical image combined with the microvasculature information for lesion tissue, a spatial map of chemotherapy accessibility to the lesion; and
generating the combined image comprising the estimated spatial map of chemotherapy accessibility to the lesion superimposed onto the medical image.

12. The apparatus of any one of claims **1-11** wherein the biopsy sample is obtained from a lesion of the medical subject, the information generated from the biopsy sample includes radiation absorption characteristic of lesion tissue for radiation emitted by a brachytherapy radiation seed, and the combining comprises:
computing, based on image features of the lesion in the medical image combined with the radiation absorption characteristic of lesion tissue, an optimal placement of brachytherapy radiation seeds respective to the lesion; and
generating the combined image comprising the optimal placement of brachytherapy radiation seeds superimposed onto the medical image.

13. A method comprising:
spatially registering a biopsy sample (S_{R}, S₀, S₁, S₂, S₃, S₄) extracted from a medical subject with a medical image **(12)** of the medical subject by identifying a region of the medical image **(12)** of the medical subject that depicts a location of the medical subject from which the biopsy sample was extracted,
combining the medical image with a graphical representation of information **(20, 22)** generated from the biopsy sample to generate a combined image in which the graphical representation is spatially delineated based on the spatial registration of the biopsy sample,
**characterized in that** the information generated from the biopsy sample includes lesion tissue growth rate, and the combining comprises:
projecting, based on the size and shape of the lesion in the medical image combined with the lesion tissue growth rate, one or more future lesion boundaries; and
wherein generating the combined image comprises generating the projected one or more future lesion boundaries superimposed onto the medical image; and
displaying the combined image on a graphical display device.

14. A non-transitory storage medium storing instructions executable by an electronic data processing device **(34)** to perform operations including
(i) spatially registering a biopsy sample (S_{R}, S₀, S₁, S₂, S₃, S₄) extracted from a medical subject with a medical image **(12)** of the medical subject by identifying a region of the medical image **(12)** of the medical subject that depicts a location of the medical subject from which the biopsy sample was extracted,
(ii) combining the medical image with a graphical representation of information **(20, 22)** generated from the biopsy sample to generate a combined image in which the graphical representation is spatially delineated based on the spatial registration of the biopsy sample; **characterized in that** the information generated from the biopsy sample includes lesion tissue growth rate, and the combining comprises:
projecting, based on the size and shape of the lesion in the medical image combined with the lesion tissue growth rate, one or more future lesion boundaries; and
wherein generating the combined image comprises generating the projected one or more future lesion boundaries superimposed onto the medical image; and
(iii) displaying the combined medical image on a graphical display device **(32).**

## Patentansprüche

1. Vorrichtung, umfassend:
eine Bildgebungs-Visualisierungs-Arbeitsstation (30), die eine grafische Anzeigevorrichtung (32) und einen elektronischen Datenprozessor enthält, wobei die Bildgebungs-Visualisierungs-Arbeitsstation konfiguriert ist, um ein Verfahren durchzuführen, das Folgendes beinhaltet:
räumliches Registrieren einer Biopsieprobe (S_{R}, S₀, S₁, S₂, S₃, S₄), die aus einem medizinischen Subjekt entnommen wurde, mit einem medizinischen Bild (12) des medizinischen Subjekts durch Identifizieren eines Bereichs des medizinischen Bildes (12) des medizinischen Subjekts, das einen Ort des medizinischen Subjekts darstellt, aus dem die Biopsieprobe entnommen wurde, durch den elektronischen Datenprozessor,
Kombinieren des medizinischen Bildes mit einer grafischen Darstellung von Informationen (20, 22), die von der Biopsieprobe erzeugt wurden, um ein kombiniertes Bild zu erzeugen, in dem die grafische Darstellung basierend auf der räumlichen Registrierung der Biopsieprobe durch den elektronischen Datenprozessor räumlich abgegrenzt ist,
**dadurch gekennzeichnet, dass**
die von der Biopsieprobe erzeugten Informationen beinhalten die Wachstumsrate des Läsionsgewebes, und das Kombinieren umfasst:
Projizieren, basierend auf der Größe und Form der Läsion in dem medizinischen Bild kombiniert mit der Wachstumsrate des Läsionsgewebes, einer oder mehrerer zukünftiger Läsionsgrenzen; und
wobei das Erzeugen des kombinierten Bildes das Erzeugen der projizierten einen oder mehreren zukünftigen Läsionsgrenzen umfasst, die dem medizinischen Bild überlagert sind; und
Anzeigen des kombinierten Bildes auf dem grafischen Anzeigegerät der Bildgebungs-Visualisierungs-Arbeitstation.

2. Vorrichtung nach Anspruch 1, wobei die Biopsieprobe aus einer Läsion des medizinischen Subjekts gewonnen wird und wobei:
das räumliche Registrieren die Biopsieprobe einer Darstellung der Läsion im medizinischen Bild zuordnet; und
das kombinierte Bild die grafische Darstellung von Informationen zuordnet, die aus der Biopsieprobe erzeugt wurden, mit der Darstellung der Läsion in dem medizinischen Bild.

3. Vorrichtung nach Anspruch 2, wobei das Kombinieren mindestens eines umfasst von:
Markieren der Darstellung der Läsion in dem medizinischen Bild mit den Informationen, die aus der aus der Läsion extrahierten Biopsieprobe erzeugt werden, und
Falschfärbung oder Hervorhebung der Darstellung der Läsion im medizinischen Bild mit einer Farbe oder Hervorhebung, die durch die aus der Biopsieprobe erzeugten Informationen bestimmt wird.

4. Vorrichtung nach Anspruch 1, wobei die Biopsieprobe einen Abschnitt von extrahiertem Gewebe umfasst und die räumliche Registrierung umfasst:
Identifizieren eines Referenzmerkmals in dem medizinischen Bild; und
Lokalisieren des extrahierten Gewebes in dem medizinischen Bild basierend auf dem Referenzmerkmal.

5. Vorrichtung nach Anspruch 4, wobei das Referenzmerkmal eine Läsionsgrenze umfasst.

6. Vorrichtung nach Anspruch 4, wobei das Referenzmerkmal eine Biopsie-Narbe umfasst.

7. Vorrichtung nach Anspruch 1, wobei die Biopsieprobe unter Verwendung eines interventionellen Instruments gewonnen wird und ein interventionelles Bild (60) des Eingriffsinstruments erfasst wird, das positioniert ist, um die Biopsieprobe zu erhalten, und die räumliche Registrierung umfasst:
räumliches Registrieren des medizinischen Bildes und des interventionellen Bildes; und
Lokalisieren der Biopsieprobe in dem medizinischen Bild basierend auf dem räumlich registrierten interventionellen Bild.

8. Vorrichtung nach einem der Ansprüche 1 bis 7, wobei das Kombinieren umfasst:
Definieren eines Bereichs (90, 92, 94, 96, 100) des medizinischen Bildes (12), der einen Abschnitt des medizinischen Subjekts darstellt, aus dem die Biopsieprobe gemäß der räumlichen Registrierung entnommen wurde; und
Modifizieren des Bereichs des medizinischen Bildes, um den Bereich abzugrenzen und die von der Biopsieprobe erzeugten Informationen anzuzeigen.

9. Vorrichtung nach Anspruch 8, wobei die Biopsieprobe eine Reihe von Biopsieproben (S₀, S₁, S₂, S₃, S₄) umfasst, die aus unterschiedlichen Tiefen in einer Läsion extrahiert wurden, und das Definieren umfasst:
Definieren des Bereichs (90, 92, 94, 96) für jede Biopsieprobe als Isokonturhülle entsprechend der Tiefe der Biopsieprobe in der Läsion innerhalb einer äußeren Hülle, die auf der Größe und Form der Läsion basiert und wobei der definierte Bereich ein zusammenhängender Bereich ist.

10. Vorrichtung nach Anspruch 8, wobei das Definieren umfasst:
Identifizieren einer Bildeigenschaft am Ort des medizinischen Bildes (12), das räumlich mit der Biopsieprobe registriert ist; und
Definieren des Bereichs des medizinischen Bildes als einen oder mehrere Abschnitte (100) des medizinischen Bildes mit der identifizierten Bildeigenschaft.

11. Vorrichtung nach einem der Ansprüche 1 bis 10, wobei die Biopsieprobe aus einer Läsion des medizinischen Subjekts erhalten wird, die aus der Biopsieprobe erzeugten Informationen Mikrovaskulaturinformationen für Läsionsgewebe enthalten und das Kombinieren umfasst:
Schätzen, basierend auf der Struktur der Läsion in dem medizinischen Bild kombiniert mit den Mikrovaskulaturinformationen für Läsionsgewebe, einer räumlichen Karte der Chemotherapie-Zugänglichkeit zur Läsion; und
Erzeugen des kombinierten Bildes, das die geschätzte räumliche Karte der Chemotherapie-Zugänglichkeit zu der Läsion umfasst, die dem medizinischen Bild überlagert ist.

12. Vorrichtung nach einem der Ansprüche 1 bis 11, wobei die Biopsieprobe aus einer Läsion des medizinischen Subjekts gewonnen wird, die aus der Biopsieprobe erzeugten Informationen eine Strahlungsabsorptionscharakteristik des Läsionsgewebes für Strahlung enthalten, die von einem Brachytherapie-Bestrahlungsseed emittiert wird, und das Kombinieren umfasst:
Berechnen, basierend auf Bildmerkmalen der Läsion in dem medizinischen Bild kombiniert mit der Strahlungsabsorptionscharakteristik des Läsionsgewebes, einer optimalen Platzierung von Brachytherapie-Bestrahlungsseed bezüglich der Läsion; und
Erzeugen des kombinierten Bildes, das die optimale Platzierung von Brachytherapie-Betrahlungsseed umfasst, die dem medizinischen Bild überlagert sind.

13. Verfahren, umfassend:
räumliches Registrieren einer Biopsieprobe (S_{R}, S₀, S₁, S₂, S₃, S₄), die aus einem medizinischen Subjekt entnommen wurde, mit einem medizinischen Bild (12) des medizinischen Subjekts durch Identifizieren eines Bereichs des medizinischen Bildes (12) des medizinischen Subjekts, das einen Ort des medizinischen Subjekts darstellt, aus dem die Biopsieprobe entnommen wurde,
Kombinieren des medizinischen Bildes mit einer grafischen Darstellung von Informationen (20, 22), die aus der Biopsieprobe erzeugt wurden, um ein kombiniertes Bild zu erzeugen, in dem die grafische Darstellung basierend auf der räumlichen Registrierung der Biopsieprobe räumlich abgegrenzt ist,
**dadurch gekennzeichnet, dass**
die von der Biopsieprobe erzeugten Informationen die Wachstumsrate des Läsionsgewebes enthalten, und das Kombinieren umfasst:
Projizieren, basierend auf der Größe und Form der Läsion in dem medizinischen Bild kombiniert mit der Wachstumsrate des Läsionsgewebes, einer oder mehrerer zukünftiger Läsionsgrenzen; und
wobei das Erzeugen des kombinierten Bildes das Erzeugen der projizierten einen oder mehreren zukünftigen Läsionsgrenzen umfasst, die dem medizinischen Bild überlagert sind; und
Anzeigen des kombinierten Bildes auf einem grafischen Anzeigegerät.

14. Nichtflüchtiges Speichermedium, das Anweisungen speichert, die durch eine elektronische Datenverarbeitungsvorrichtung (34) ausführbar sind, um Operationen durchzuführen, einschließlich
(i) räumliches Registrieren einer Biopsieprobe (S_{R}, S₀, S₁, S₂, S₃, S₄), die aus einem medizinischen Subjekt entnommen wurde, mit einem medizinischen Bild (12) des medizinischen Subjekts durch Identifizieren eines Bereichs des medizinischen Bildes (12) des medizinisches Subjekt, das einen Ort des medizinischen Subjekts darstellt, aus dem die Biopsieprobe entnommen wurde,
(ii) Kombinieren des medizinischen Bildes mit einer grafischen Darstellung von Informationen (20, 22), die aus der Biopsieprobe erzeugt wurden, um ein kombiniertes Bild zu erzeugen, in dem die grafische Darstellung basierend auf der räumlichen Registrierung der Biopsieprobe räumlich abgegrenzt ist;
**dadurch gekennzeichnet, dass**
die aus der Biopsieprobe generierten Informationen die Wachstumsrate des Läsionsgewebes enthalten, und das Kombinieren umfasst:
Projizieren, basierend auf der Größe und Form der Läsion in dem medizinischen Bild kombiniert mit der Wachstumsrate des Läsionsgewebes, einer oder mehrerer zukünftiger Läsionsgrenzen; und
wobei das Erzeugen des kombinierten Bildes das Erzeugen der projizierten einen oder mehreren zukünftigen Läsionsgrenzen umfasst, die dem medizinischen Bild überlagert sind; und
(iii) Anzeigen des kombinierten medizinischen Bildes auf einer grafischen Anzeigevorrichtung (32).

## Revendications

1. Appareil comprenant:
une station de travail de visualisation d'imagerie (30) comprenant un dispositif d'affichage graphique (32) et un processeur de données électroniques, le station de travail de visualisation d'imagerie étant configurée pour exécuter un procédé consistant à:
recaler spatialement un échantillon de biopsie (S_{R}, S₀, S₁, S₂, S₃, S₄) extrait d'un sujet médical avec une image médicale (12) du sujet médical en identifiant une région de l'image médicale (12) du sujet médical qui représente un emplacement du sujet médical à partir duquel l'échantillon de biopsie a été extrait, par le processeur de données électroniques,
combiner l'image médicale avec une représentation graphique d'informations (20, 22) générées à partir de l'échantillon de biopsie pour générer une image combinée dans laquelle la représentation graphique est délimitée spatialement sur la base du recalage spatial de l'échantillon de biopsie par le processeur de données électroniques,
**caractérisé en ce que**
les informations générées à partir de l'échantillon de biopsie comprennent le taux de croissance du tissu de la lésion, et la combinaison consistant à:
projecter, sur la base de la taille et de la forme de la lésion dans l'image médicale combinée au taux de croissance du tissu de la lésion, une ou plusieurs limites futures de lésion; et
où la génération de l'image combinée comprend la génération de l'une ou des plusieurs limites futures de lésion projetées superposées sur l'image médicale; et
afficher l'image combinée sur le dispositif d'affichage graphique de la station de travail de visualisation d'imagerie.

2. Appareil selon la revendication 1, dans lequel l'échantillon de biopsie est obtenu à partir d'une lésion du sujet médical et dans lequel:
le recalage spatial associe l'échantillon de biopsie à une représentation de la lésion dans l'image médicale; et
l'image combinée associe la représentation graphique des informations générées à partir de l'échantillon de biopsie avec la représentation de la lésion dans l'image médicale.

3. Appareil selon la revendication 2, dans lequel la combinaison comprend au moins l'un parmi:
l'étiquetage de la représentation de la lésion dans l'image médicale avec les informations générées à partir de l'échantillon de biopsie extrait de la lésion, et
la fausse coloration ou la mise en évidence de la représentation de la lésion dans l'image médicale avec une couleur ou une mise en évidence déterminée par les informations générées à partir de l'échantillon de biopsie.

4. Appareil selon la revendication 1, dans lequel l'échantillon de biopsie comprend une section de tissu extrait, et le recalage spatial consiste à:
identifier une caractéristique de référence dans l'image médicale; et
localiser le tissu extrait dans l'image médicale sur la base de la caractéristique de référence.

5. Appareil selon la revendication 4, dans lequel la caractéristique de référence comprend une limite de lésion.

6. Appareil selon la revendication 4 dans lequel la caractéristique de référence comprend une cicatrice de biopsie.

7. Appareil selon la revendication 1, dans lequel l'échantillon de biopsie est obtenu à l'aide d'un instrument interventionnel et une image interventionnelle (60) est acquise de l'instrument interventionnel positionné pour obtenir l'échantillon de biopsie, et le recalage spatial consiste à:
recaler spatialement l'image médicale et l'image interventionnelle; et
localiser l'échantillon de biopsie dans l'image médicale sur la base de l'image interventionnelle recalée spatialement.

8. Appareil selon l'une quelconque des revendications 1 à 7, dans lequel la combinaison consiste à:
définir une région (90, 92, 94, 96, 100) de l'image médicale (12) représentant une partie du sujet médical à partir de laquelle l'échantillon de biopsie a été extrait selon le recalage spatial; et
modifier la région de l'image médicale pour délimiter la région et indiquer les informations générées à partir de l'échantillon de biopsie.

9. Appareil selon la revendication 8, dans lequel l'échantillon de biopsie comprend une ligne d'échantillons de biopsie (S₀, S₁, S₂, S₃, S₄) extraits à différentes profondeurs dans une lésion et la définition consiste à:
pour chaque échantillon de biopsie, définir la région (90, 92, 94, 96) comme une coque d'isocontour correspondant à la profondeur de l'échantillon de biopsie dans la lésion à l'intérieur d'une coque externe qui est basée sur la taille et la forme de la lésion et où la région définie est une région contiguë.

10. Appareil selon la revendication 8, dans lequel la définition consiste à:
identifier une caractéristique d'image à l'emplacement de l'image médicale (12) spatialement recalée avec l'échantillon de biopsie; et
définir la région de l'image médicale comme une ou plusieurs parties (100) de l'image médicale ayant la caractéristique d'image identifiée.

11. Appareil selon l'une quelconque des revendications 1 à 10, dans lequel l'échantillon de biopsie est obtenu à partir d'une lésion du sujet médical, les informations générées à partir de l'échantillon de biopsie comprennent des informations microvasculaires pour le tissu de la lésion, et la combinaison consiste à:
estimer, sur la base de la structure de la lésion dans l'image médicale combinée aux informations microvasculaires pour le tissu de la lésion, une carte spatiale de l'accessibilité de la chimiothérapie à la lésion; et
générer l'image combinée comprenant la carte spatiale estimée d'accessibilité de la chimiothérapie à la lésion superposée sur l'image médicale.

12. Appareil selon l'une quelconque des revendications 1 à 11, dans lequel l'échantillon de biopsie est obtenu à partir d'une lésion du sujet médical, les informations générées à partir de l'échantillon de biopsie comprennent la caractéristique d'absorption de rayonnement du tissu de la lésion pour le rayonnement émis par un grain radioactif de curiethérapie, et la combinaison consiste à :
calculer, sur la base de caractéristiques d'image de la lésion dans l'image médicale combinées à la caractéristique d'absorption de rayonnement du tissu de la lésion, un placement optimal des grains radioactifs de curiethérapie par rapport à la lésion; et
générer l'image combinée comprenant le placement optimal des grains radioactifs de curiethérapie superposée sur l'image médicale.

13. Procédé consistant à:
recaler spatialement un échantillon de biopsie (S_{R}, S₀, S₁, S₂, S₃, S₄) extrait d'un sujet médical avec une image médicale (12) du sujet médical en identifiant une région de l'image médicale (12) du sujet médical qui représente un emplacement du sujet médical à partir duquel l'échantillon de biopsie a été extrait,
combiner l'image médicale avec une représentation graphique d'informations (20, 22) générées à partir de l'échantillon de biopsie pour générer une image combinée dans laquelle la représentation graphique est délimitée spatialement sur la base du recalage spatial de l'échantillon de biopsie,
**caractérisé en ce que**
les informations générées à partir de l'échantillon de biopsie comprennent le taux de croissance du tissu de la lésion, et la combinaison consiste à:
projecter, sur la base de la taille et de la forme de la lésion dans l'image médicale combinée au taux de croissance du tissu de la lésion, une ou plusieurs limites futures de lésion; et
où la génération de l'image combinée comprend la génération de l'une ou des plusieurs limites futures de lésion projetées superposées sur l'image médicale; et
afficher l'image combinée sur le dispositif d'affichage graphique.

14. Support de stockage non transitoire stockant des instructions exécutables par un dispositif de traitement de données électroniques (34) pour effectuer des opérations consistant à
(i) recaler spatialement un échantillon de biopsie (S_{R}, S₀, S₁, S₂, S₃, S₄) extrait d'un sujet médical avec une image médicale (12) du sujet médical en identifiant une région de l'image médicale (12) du sujet médical qui représente un emplacement du sujet médical à partir duquel l'échantillon de biopsie a été extrait,
(ii) combiner l'image médicale avec une représentation graphique d'informations (20, 22) générées à partir de l'échantillon de biopsie pour générer une image combinée dans laquelle la représentation graphique est délimitée spatialement sur la base du recalage spatial de l'échantillon de biopsie;
**caractérisé en ce que**
les informations générées à partir de l'échantillon de biopsie comprennent le taux de croissance du tissu de la lésion, et la combinaison consiste à:
projecter, sur la base de la taille et de la forme de la lésion dans l'image médicale combinée au taux de croissance du tissu de la lésion, une ou plusieurs limites futures de lésion; et
où la génération de l'image combinée comprend la génération de l'une ou des plusieurs limites futures de lésion projetées superposées sur l'image médicale; et
(iii) afficher l'image médicale combinée sur le dispositif d'affichage graphique (32).
